# EUROPÄISCHE PATENTANMELDUNG

(11) **EP 1 188 732 A2**
(43) Veröffentlichungstag der Anmeldung: **20.03.2002**
(21) Anmeldenummer: 01120764.4
(22) Anmeldetag: 07.09.2001
(51) Int. Cl.: C07B 61/00, C07B 37/12, C07D 403/04, C07D 401/04, C07D 405/04, C07D 207/08

(54) **Kombinatorische Herstellung einer Substanzbibliothek unter Abtrennung einer zwitterionischen Verbindung durch Fällung**

(30) Priorität: 13.09.2000 DE 10045530
(71) Anmelder: BASF AKTIENGESELLSCHAFT, 67056 Ludwigshafen (DE)
(72) Erfinder: Tietze, Lutz F., Prof. Dr., 37077 Göttingen (DE); Evers, Holger, Dr., Madison, WI 53705 (US); Töpken, Enno, 37073 Göttingen (DE)
(74) Vertreter: Isenbruck, Günter, Dr.

(57) **Zusammenfassung**

Verfahren zur kombinatorischen Herstellung einer Substanzbibliothek aus n verschiedenen Verbindungen in einem Array aus n verschiedenen Reaktionsansätzen an n räumlich getrennten Reaktionsorten, bei dem an jedem der n Reaktionsorte
a) aus jeweils m Reaktanten in einer Reaktion oder Reaktionssequenz eine zwitterionische Verbindung in einem polaren Lösungsmittel gelöst erhalten wird,
b) die zwitterionische Verbindung durch Zugabe eine unpolaren Lösungsmittels als Feststoff gefällt, isoliert und damit in reiner Form erhalten wird,
c) gegebenenfalls die zwitterionische Verbindung weiter umgesetzt wird, wobei sich jeder der n Reaktionsansätze von jedem der n - 1 übrigen Reaktionsansätzen in mindestens einem der m Reaktanten unterscheidet.

## Beschreibung

Die Erfindung betrifft ein Verfahren zur Herstellung einer Substanzbibliothek aus n verschiedenen Verbindungen.

Kombinatorische Synthesen werden derzeit sowohl in flüssiger als auch an fester Phase durchgeführt. Beide Verfahren weisen Nachteile auf. Bei der Festphasen-Synthese müssen zusätzlich zwei Stufen durchgeführt werden, nämlich die Anknüpfung an und die Abspaltung von einem polymeren Träger. Darüber hinaus sind viele Reaktionen derzeit an fester Phase nicht oder nur eingeschränkt möglich. Bei der Flüssigphasen-Synthese ist die Abtrennung von Reagenzüberschüssen und Reinigung der Reaktionsprodukte problematisch.

Aufgabe der Erfindung ist, ein Verfahren zur kombinatorischen Synthese einer Substanzbibliothek bereitzustellen, das die Vorteile der herkömmlichen Fest- und Flüssigphasen-Synthese auf sich vereinigt, aber deren Nachteile weitgehend vermeidet.

Gelöst wird die Aufgabe durch ein Verfahren zur kombinatorischen Herstellung einer Substanzbibliothek aus n verschiedenen Verbindungen in einem Array aus n verschiedenen Reaktionsansätzen an n räumlich getrennten Reaktionsorten, bei dem an jedem der n Reaktionsorte
(a) aus jeweils m Reaktanten in einer Reaktion oder Reaktionssequenz eine zwitterionische Verbindung in einem polaren Lösungsmittel gelöst erhalten wird,
(b) die zwitterionische Verbindung durch Zugabe eines unpolaren Lösungsmittels als Feststoff gefällt, isoliert und damit in reiner Form erhalten wird,
(c) gegebenenfalls die zwitterionische Verbindung weiter umgesetzt wird, wobe sich jeder der n Reaktionsansätze von jedem der n-1 übrigen Reaktionsansätzen in mindestens einem der m Reaktanten unterscheidet.

Bei dem erfindungsgemäßen Verfahren wird als Zwischen- oder Endprodukt eine zwitterionische Verbindung (ein Betain) gebildet, die in wässrigen, wässrig-organischen oder polaren organischen Medien löslich ist, in unpolaren Medien jedoch unlöslich ist. Dadurch kann die im Verlauf einer Reaktion oder Reaktionssequenz gebildete zwitterionische Verbindung problemlos aus dem Reaktionsgemisch abgetrennt werden, indem sie aus Lösung in einem polaren Lösungsmittel durch Zugabe eines unpolaren organischen Lösungsmitels als Feststoff gefällt und isoliert wird.

Geeignete polare Lösungsmittel sind alle Lösungsmittel, in dem die zwitterionischen Verbindungen löslich sind. Beispiele sind Wasser und Alkohole wie Methanol und Ethanol oder deren Gemische. Geeignete unpolare Lösungsmittel sind solche, die mit den polaren Lösungsmitteln zumindest teilweise mischbar sind. Ein Beispiel ist Diethylether. Durch Zugabe des unpolaren Lösungsmittels zu der Lösung der zwitterionischen Verbindung wird ein Lösungsmittelgemisch mit geringerer Polarität erhalten, worauf die zwitterionische Verbindung ausfällt. Der gefällte Feststoff wird dadurch in hoher Reinheit erhalten.

Die zwitterionische Verbindung wird in einem polaren Lösungsmittel gelöst erhalten. Die zwitterionische Verbindung kann bereits in dem polaren Lösungsmittel gebildet werden, oder es kann die bereits gebildete Verbindung in einem Aufarbeitungsschritt in dem polaren Lösungsmittel gelöst werden.

In einer Ausführungsform des erfindungsgemäßen Verfahrens werden als m Reaktanten ein geschütztes Aminoaldehyd der allgemeinen Formel 1, 2 oder 3 mit einem Enolether der allgemeinen Formel 5 und einer 1,3-Dicarbonylverbindung 4 in untenstehender Reaktionssequenz zu einem Betain der allgemeinen Formel 6, 7 bzw. 8 als zwitterionischer Verbindung umgesetzt:

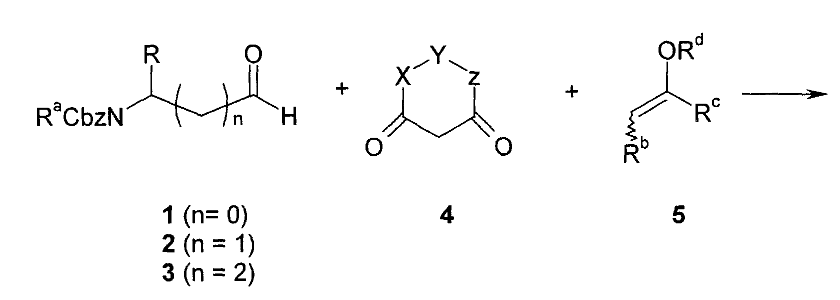

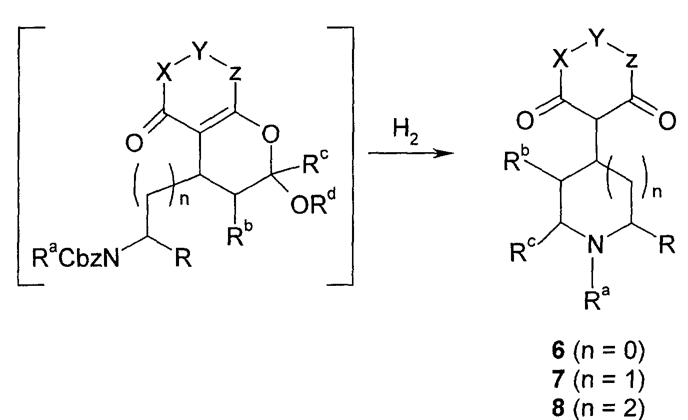

Darin bedeuten Cbz eine Benzyloxycarbonylgruppe und R^{d} eine Benzyl- oder Trimethylsilylgruppe.

Durch Kondensation des geschützten Aminoaldehyds 1, 2 oder 3 mit der 1,3-Dicarbonylverbindung 4 und nachfolgender Hetero-Diels-Alder-Reaktion des gebildeten Kondensationsproduktes mit dem Enolether 5 wird ein Acetal als Zwischenprodukt erhalten. Das Acetal wird anschließend durch hydrogenolytische Abspaltung der Benzyl-Schutzgruppe in den feien Aldehyd bzw., falls R^{c} ≠ H ist, in das freie Keton überführt. Durch hydrogenolytische Abspaltung der Benzyloxycarbonylgruppe wird darüber hinaus eine freie Aminfunktion gebildet. Durch intramolekulare Kondensation des Aldehyds mit dem Amin wird in situ ein cyclisches Enamin/Imin gebildet, das unter den Bedingungen der Hydrogenolyse zu dem entsprechenden gesättigten Stickstoff-Heterocyclus 6, 7 bzw. 8 reduziert wird.

Die Kondensation des geschützten Aminoaldehyds 1, 2 oder 3 mit der 1,3-Dicarbonylverbindung 4 wird bevorzugt in Gegenwart eines sauren oder basischen Katalysators wie Ethylendiammoniumdiacetat und/oder eines wasserentziehenden Mittels wie Trialkylorthoformiat durchgeführt. Dabei wird bevorzugt in Toluol als Lösungsmittel und unter Schutzgas-Atmosphäre gearbeitet.

Die Hydrogenolyse kann an jedem geeigneten Hydrierungskatalysator, beispielsweise Palladium auf Aktivkohle, durchgeführt werden. Dabei wird bevorzugt in Methanol als Lösungsmittel gearbeitet, wobei das Betain 6, 7 bzw. 8 in Methanol als polarem Lösungsmittel erhalten wird. Bevorzugt wird das Betain durch Zugabe von Diethylether als unpolarem Lösungsmittel gefällt. Die Lösung des Betains kann vor dem Fällungsschritt durch Abdestillieren von Methanol eingeengt werden.

Die Betaine 6, 7 bzw. 8 werden mit einer Reinheit von vorzugsweise 80 bis 100 mol.-% isoliert.

Geeignete Verbindungen 1 - 5 sind beispielsweise solche, die pharmakophore Gruppen enthalten, wie Barbitursäuren und Hydroxycumarine, sowie weitere 1,3-Dicarbonylverbindungen, welche mit Aminoaldehyden, herstellbar aus natürlichen und synthetischen Aminosäuren und Aminoalkoholen, und verschiedensten Benzylenolethern zur Reaktion gebracht werden können.

Bevorzugte geschützte Aminoaldehyde sind die alpha-, beta- und gamma-Aminoaldehyde der allgemeinen Formeln 1a bis 1e: 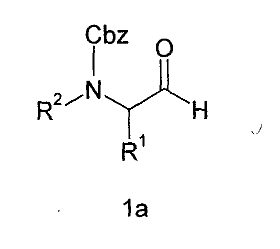 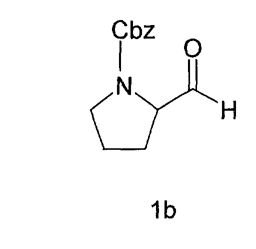 mit
- R¹: H, Alkyl, CH₂OH, CH₂SH,
- R²: H, Alkyl
und Alkyl = Me, Et, n-Pr, i-Pr, n-Bu, sek-Bu, i-Bu oder Pentyl 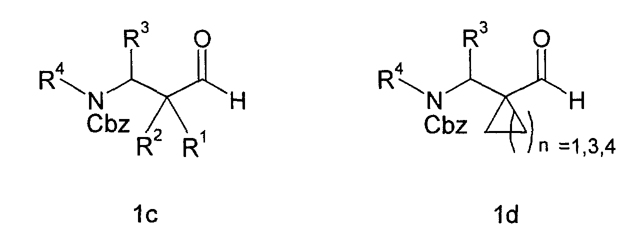 mit
- R¹, R²,: gleich oder verschieden, H, Alkyl,
- R³, R⁴,: gleich oder verschieden, H, Alkyl,
und Alkyl = Me, Et, n-Pr, i-Pr, n-Bu, sek-Bu, i-Bu oder Pentyl 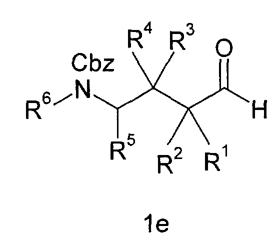 mit
- R¹, R², R³, R⁴,: gleich oder verschieden, H, Alkyl,
- R⁵, R⁶,: gleich oder verschieden, H, Alkyl,
und Alkyl = Me, Et, n-Pr, n-Bu, sek-Bu, i-Bu oder Pentyl

Bevorzugte Enolether sind diejenigen der allgemeinen Formeln 2a bis 2d: 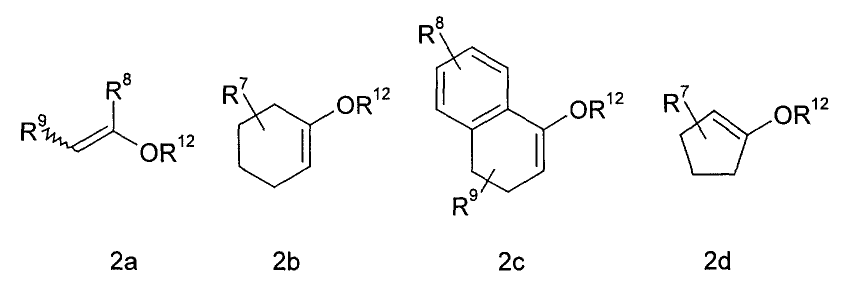 mit
- R⁷, R⁸, R⁹,: gleich oder verschieden = H, Alkyl, Bn, Ph-C₂H₄,CH₂OR, CH₂SR, CH₂NHAc, CH₂CH₂OR, CH₂CH₂SR, CH₂CH₂NHAc, und
- R⁹: zusätzlich Ph,
- R¹²: Benzyl oder - Si (CH₃)₃,
und Alkyl = Me, Et, n-Pr, i-Pr,n-Bu, sek-Bu, i-Bu oder Pentyl

Bevorzugte 1,3-Dicarbonylverbindungen sind diejenigen der allgemeinen Formeln 3a und 3b: 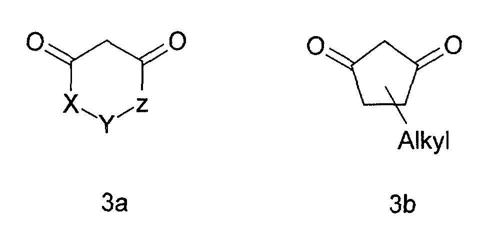 mit
- X, Z,: gleich oder verschieden, NH, NAlkyl, CH₂, CMe₂, O, S, und
- Y: CH₂, CMe₂, C=O, C=S, oder
- X-Y-Z: NH-CR¹⁰=CR¹¹, NAlkyl-CR¹⁰=CR¹¹,
O-CR¹⁰=CR¹¹, NAlkyl-CHR¹-CHR²,
mit R¹⁰ und R¹¹ gleich oder verschieden = Alkyl, unsubstituiertes oder mit OH, OAlkyl, NH₂, NHAlkyl oder N(Alkyl)₂ substituiertes Phenyl, unsubstituiertes oder mit OH, OAlkyl, NH₂, NHAlkyl oder N(Alkyl)₂ substituiertes 1,3-Butadien-1,3-dienyl, wobei im zuletzt genannten Fall R¹⁰ und R¹¹ Teile des gleichen Ringsystems sind,
und Alkyl = Me, Et, n-Pr, i-Pr, n-Bu, sek-Bu, i-Bu oder Pentyl.

Vorstehend bedeuten Me = Methyl, Et = Ethyl, Pr = Propyl, Bu = Butyl, Ph = Phenyl und Bn=Benzyl.

Nachstehend sind einige Beispielreaktionen aufgeführt.

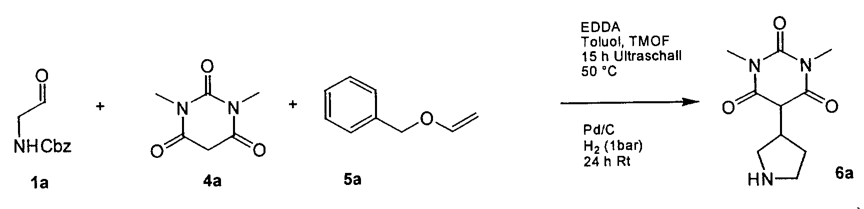

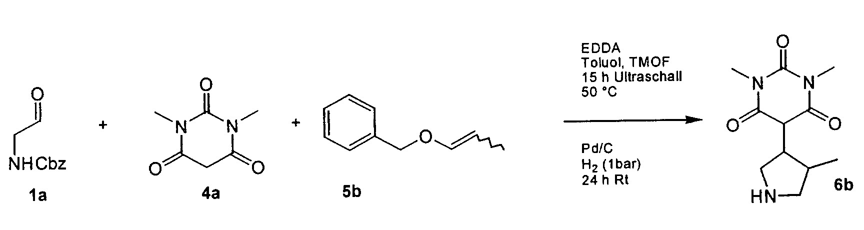

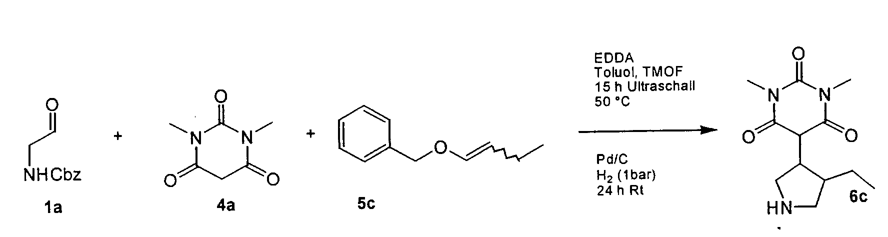

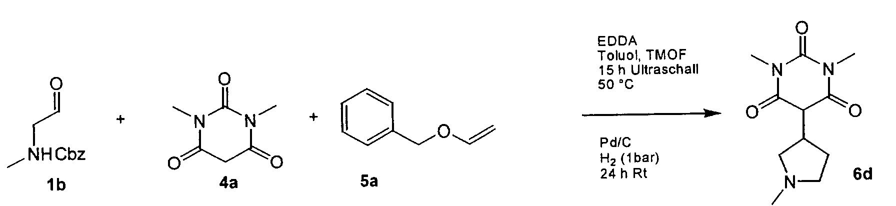

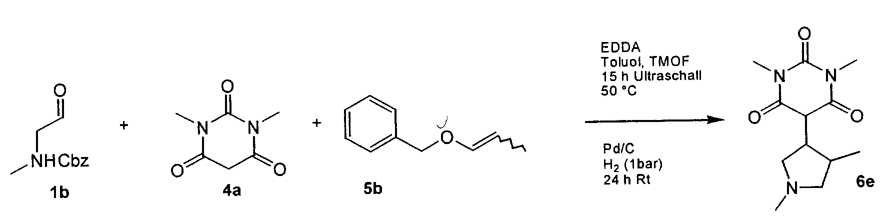

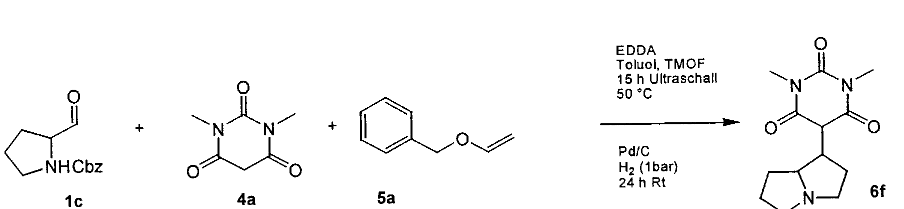

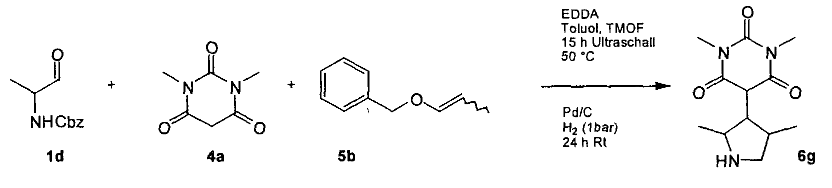

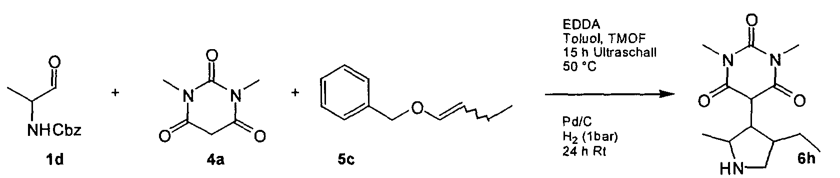

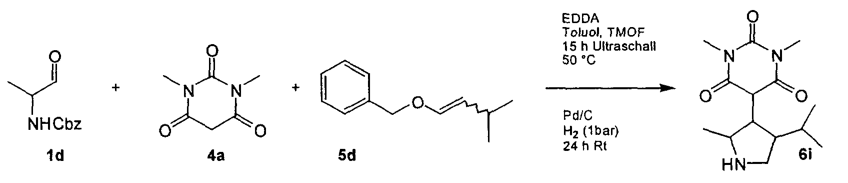

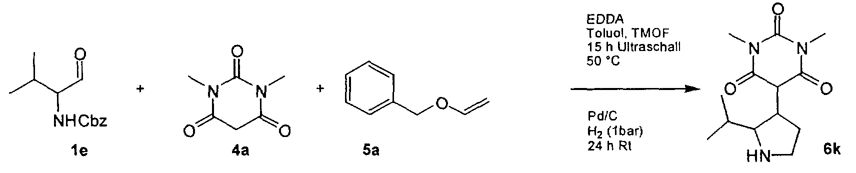

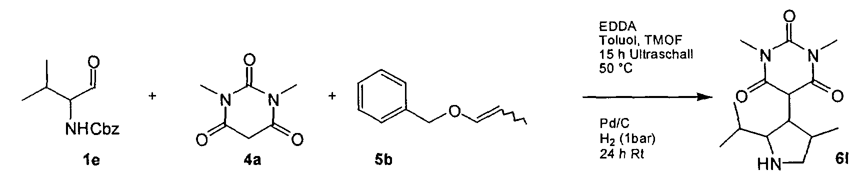

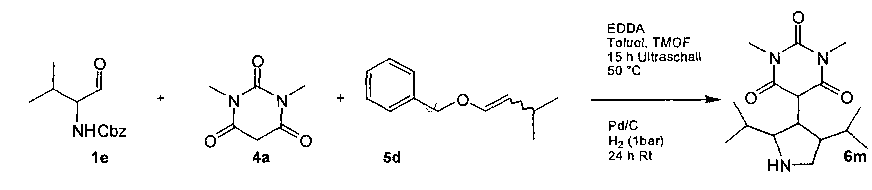

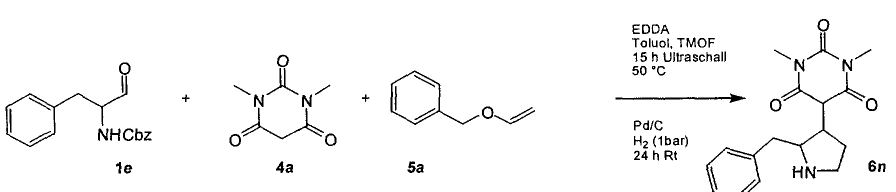

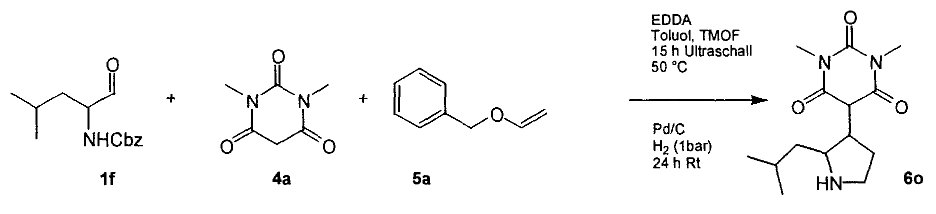

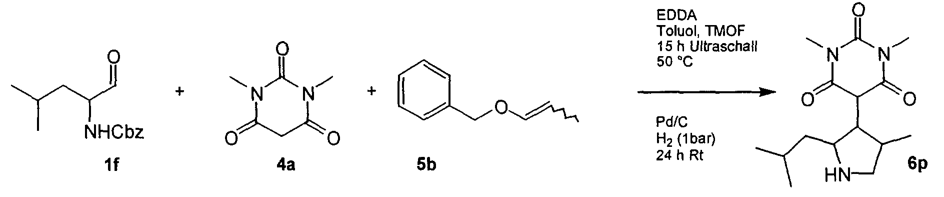

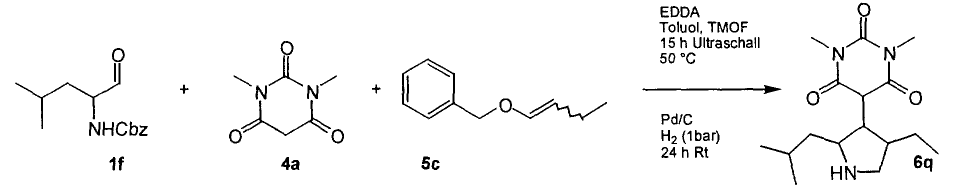

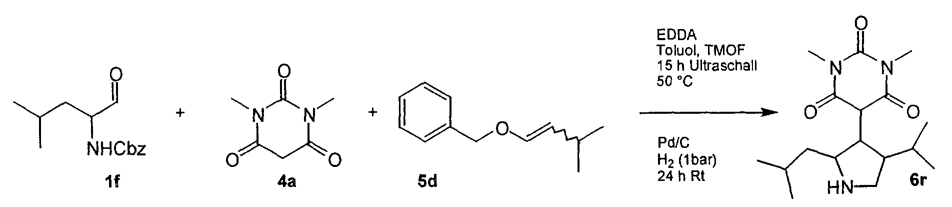

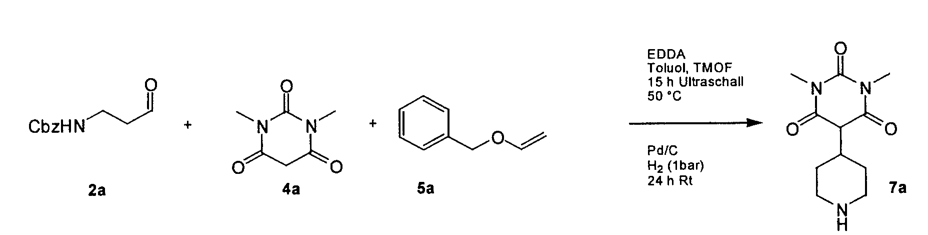

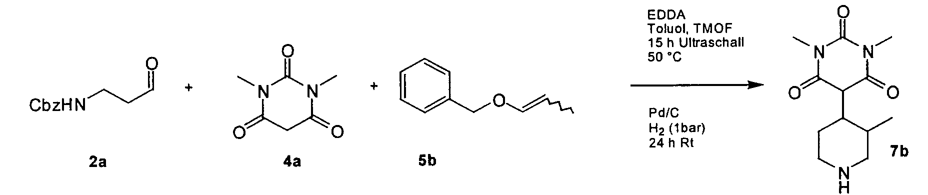

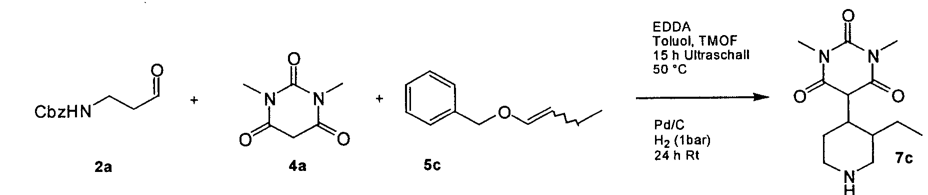

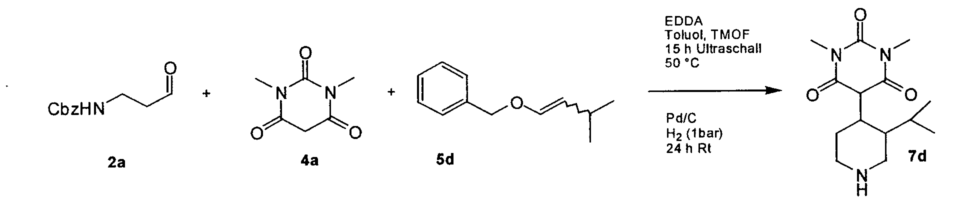

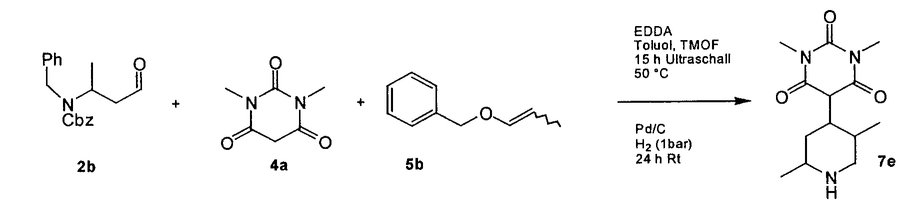

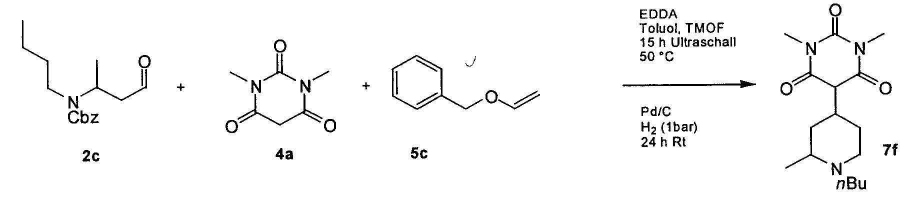

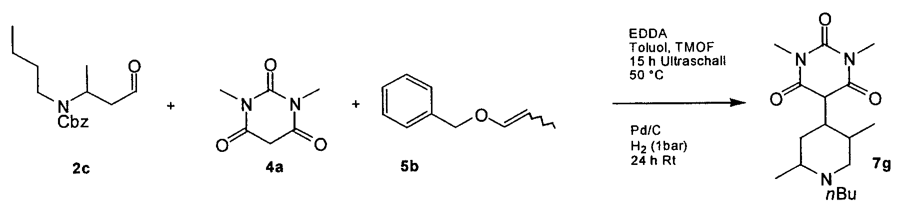

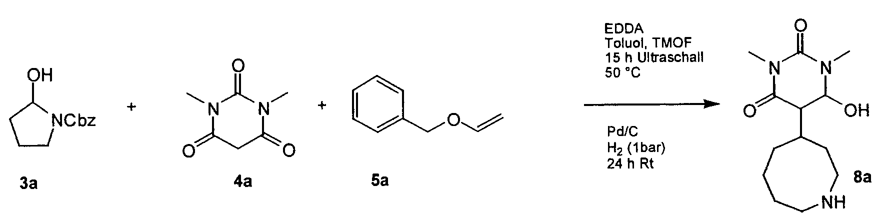

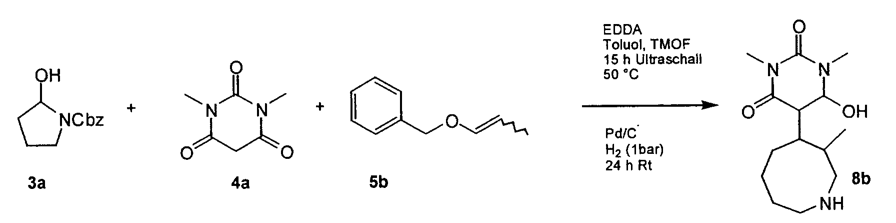

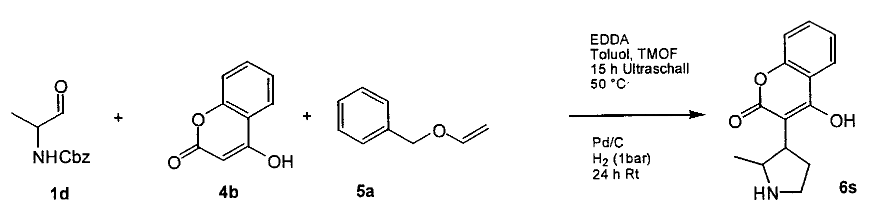

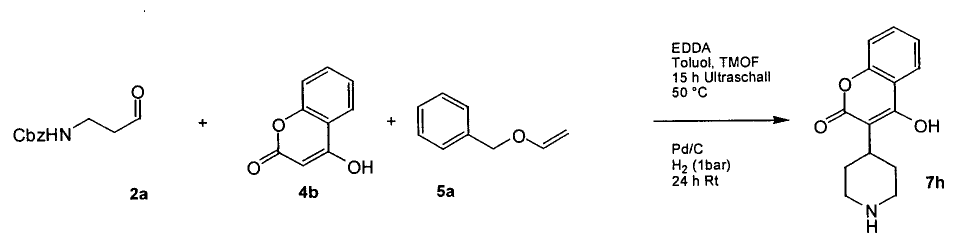

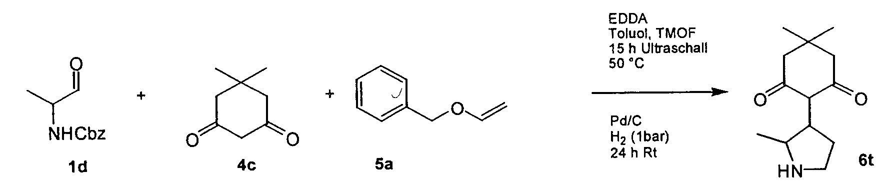

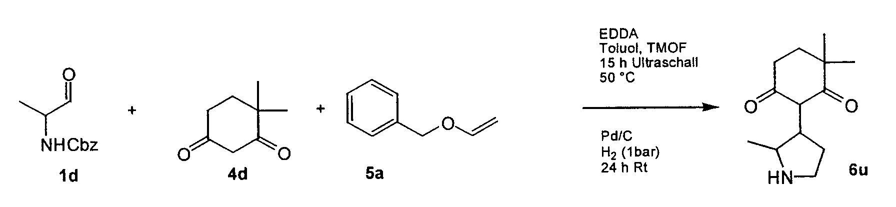

Durch die Kombination der Aminoaldehyde 1, 2 bzw. 3 mit den Dicarbonylverbindungen 5 und den Enolethern 4 kann eine sehr große Zahl unterschiedlicher Betaine 6, 7 bzw. 8 erhalten werden.

Die isolierten zwitterionischen Verbindungen können weiter umgesetzt werden.

Die Reaktionen werden in n verschiedenen Reaktionsansätzen an n räumlich getrennten Reaktionsorten durchgeführt, wobei n mindestens 2 beträgt. Pro Reaktionsansatz wird mindestens einer der m Reaktanten variiert, so daß sich jeder der n Reaktionsansätze von jedem der n -1 übrigen Reaktionsansätze in mindestens einem der m Reaktanten unterscheidet. Beispielsweise ergibt die Kombination von 10 verschiedenen Aminoaldehyden 1, 2 oder 3 mit 10 verschiedenen Enolethern 5 und 10 verschiedenen 1,3-Dicarbonylverbindungen 4 insgesamt 1000 verschiedene Reaktionsansätze. Die n Reaktionen können parallel und automatisiert durchgeführt werden, wobei übliche Syntheseautomaten eingesetzt werden können. Beim automatisierten Arbeiten kann n sehr groß sein und beispielsweise bis zu 100, 1000 oder 10 000 betragen. Es können auch bestimmte Teilschritte, beispielsweise die Umsetzungsschritte, automatisiert durchgeführt werden und andere Schritte, beispielsweise die Aufarbeitungsschritte, manuell durchgeführt werden. In jedem Fall ermöglicht das problemlose Abtrennen der zwitterionischen Verbindung von dem Reaktionsgemisch als Feststoff durch Fällen und Isolieren eine erhebliche Zeitersparnis, so daß auch bei manuellem oder halbautomatisiertem Arbeiten eine große Zahl von Reaktionen, beispielsweise 10 oder 100 Reaktionen, parallel durchgeführt werden können.

Die erhaltenen Verbindungen können an geeigneten Testsystemen beispielsweise auf ihre pharmakologische Wirksamkeit und/oder ihre Wirksamkeit als Pflanzenschutzmittel geprüft werden.

Die Erfindung wird durch die nachstehenden Beispiele näher erläutert.

### Allgemeine Arbeitsvorschrift

### AAV: Domino-Knoevenagel-hetero-Diels-Alder-Reaktion mit geschützten Aminoaldehyden

1.0 Äquivalente des Cbz-geschützten Aminoaldehyds, 1.0 Äquivalente der 1,3-Dicarbonyl-Komponente, 4.0 Äquivalente des Enolethers sowie Toluol (4 ml/mmol), Trimethylorthoformiat (0.8 ml/mmol) und wenige Kristalle Ethylendiammoniumdiacetat (EDDA) wurden unter Argonatmosphäre in einem 10 ml-Druckkolben zusammengegeben. Die Reaktionsmischung wurde im Ultraschallbad 15 h bei 50-60°C beschallt (DC-Kontrolle). Nach vollständigem Umsatz wurde das Lösungsmittel am Rotationsverdampfer entfernt und der Rückstand in Methanol (4 ml/mmol) gelöst. Nach Zugabe von Palladium auf Kohle (10 %, 100 mg/mmol) rührte man 24h unter Wasserstoffatmosphäre (DC-Kontrolle). Anschließend wurde der Katalysator über wenig Celite abfiltriert, mit Methanol nachgewaschen und das Lösungsmittel erneut im Vakuum abgezogen. Den Rückstand nahm man in wenig Methanol auf und fällte das Produkt durch langsame Zugabe von Diethylether aus. Der weiße amorphe Niederschlag wurde abfiltriert und mit Diethylether nachgewaschen.

### Erhaltene Produkte

### 3-(1,3-Dimethyl-2,4,6-trioxo-hexahydro-pyrimidin-5-yl)-pyrrolidin (6a)

*N*-Cbz-Aminoacetaldehyd (24.1 mg, 125 µmol), *N,N*-Dimethylbarbitursäure (19.5 mg, 125 µmol) und Benzylvinylether (134 mg, 1.00 mmol) wurden mit 0.5 ml Toluol, 0.1 ml Trimethylorthoformiat und wenigen Kristallen Ethylendiammoniumdiacetat (EDDA) unter Argonatmosphäre in einem 10 ml-Druckkolben gemäß der AAV umgesetzt. Das Produkt wurde nach Lösen in Methanol und anschließendem Entfernen des Lösungsmittel als gelbliches Öl erhalten, welches später amorph auskristallisierte. Die per HPLC bestimmte Reinheit betrug >95 %.

^{**1**}**H-NMR** (500 MHz, CD₃OD): δ = 2.06 - 2.14, 2.18 - 2.25 (2 m, 2H, 4-H), 3.14 - 3.26 (m, 8H, 2xNMe, 5-H), 3.41 - 3.47, 3.50 - 3.56 (2m, 2H, 2-H), 3.78 - 3.88 (m, 1H, 3-H).
^{**13**}**C-NMR** (50 MHz, CD₃OD): δ = 28.02, 34.30 (NMe, C-3), 30.22 (C-4), 88.14 (C-5'), 154.5 (C-2'), 165.1 (C-4', C-6').

**C**_{**10**}**H**_{**15**}**N**_{**3**}**O**_{**3**} **(225.1), HRMS: 225.1113.**

### 3-(1,3-Dimethyl-2,4,6-trioxo-hexahydro-pyrimidin-5-yl)-4-methyl-pyrrolidin (6b)

*N-*Cbz-Aminoacetaldehyd (24.1 mg, 125 µmol), *N,N*-Dimethylbarbitursäure (19.5 mg, 125 µmol) und Benzylprop-1-enylether (148 mg, 1.00 mmol) wurden mit 0.5 ml Toluol, 0.1 ml Trimethylorthoformiat und wenigen Kristallen Ethylendiammoniumdiacetat (EDDA) unter Argonatmosphäre in einem 10 ml-Druckkolben gemäß der AAV umgesetzt. Das Produkt wurde nach Lösen in Methanol und anschließendem Entfemen des Lösungsmittel als gelbliches Öl erhalten, welches später amorph auskristallisierte. Die per HPLC bestimmte Reinheit betrug >95 %.

**MS** (70eV): *m/z* = 239.1 (100) [M⁺], 156.1 (45) [C₆H₈N₂O₃], 83.0 (100) [M⁺ - C₆H₈N₂O₃], 68.0 (73) [M⁺ - C₆H₈N₂O₃ - Methyl].

**C**_{**11**}**H**_{**17**}**N**_{**3**}**O**_{**3**} **(239.1), HRMS: 239.1269.**

### 3-(1,3-Dimethyl-2,4,6-trioxo-hexahydro-pyrimidin-5-yl)-4-ethyl-pyrrolidin (6c)

*N*-Cbz-Aminoacetaldehyd (24.1 mg, 125 µmol), *N,N*-Dimethylbarbitursäure (19.5 mg, 125 µmol) und Benzylbut-1-enylether (162 mg, 1.00 mmol) wurden mit 0.5 ml Toluol, 0.1 ml Trimethylorthoformiat und wenigen Kristallen Ethylendiammoniumdiacetat (EDDA) unter Argonatmosphäre in einem 10 ml-Druckkolben gemäß der AAV umgesetzt. Das Produkt wurde nach Lösen in Methanol und anschließendem Entfernen des Lösungsmittel als gelbliches Öl erhalten, welches später amorph auskristallisierte. Die per HPLC bestimmte Reinheit betrug 97 %.

^{**13**}**C-NMR** (50 MHz, CD₃OD): δ = 12.75, 13.55 (C-2"), 23.45, 24.15 (C-1"), 27.76, 27.78, 28.02, 28.23 (NMe), 40.64 (C-4), 44.93, 45.49 (C-3), 52.31 (C-2 oder 5), 86.93, 87.65 (C-5'), 154.6, 154.7 (C-2'), 165.0, 165.2, 165.8 (C-4', C-6').
**MS** (70eV): *m/z* = 253.2 (10) [M⁺], 156.1 (6) [C₆H₈N₂O₃], 97.1 (82) [M⁺ - C₆H₈N₂O₃], 68.0 (100) [M⁺ - C₆H₈N₂O₃ - Et].

**C**_{**12**}**H**_{**19**}**N**_{**3**}**O**_{**3**} **(253.1), HRMS: 253.1426.**

### 3-(1,3-Dimethyl-2,4,6-trioxo-hexahydro-pyrimidin-5-yl)-1-methyl-pyrrolidin (6d)

*N*-Cbz-Methylaminoacetaldehyd (25.9 mg, 125 µmol), *N,N-*Dimethylbarbitursäure (19.5 mg, 125 µmol) und Benzylvinylether (134 mg, 1.00 mmol) wurden mit 0.5 ml Toluol, 0.1 ml Trimethylorthoformiat und wenigen Kristallen Ethylendiammoniumdiacetat (EDDA) unter Argonatmosphäre in einem 10 ml-Druckkolben gemäß der AAV umgesetzt. Das Produkt wurde nach Lösen in Methanol und anschließendem Entfernen des Lösungsmittel als gelbliches Öl erhalten, welches später amorph auskristallisierte.

^{**13**}**C-NMR** (50 MHz, CD₃OD): δ = 30.77 (C-4), 28.05, 33.95, 40.40 (C-3, CONMe, NMe), 57.42, 61.07 (C-2, C-5), 90.74 (C-5'), 154.5 (C-2'), 165.1 (C-4', C-6').
**MS** (70eV): *m/z* = 239.2 (20) [M⁺], 83.0 (100) [M⁺ - C₆H₈N₂O₃].

**C**_{**11**}**H**_{**17**}**N**_{**3**}**O**_{**3**} **(239.2).**

### 3-(1,3-Dimethyl-2,4,6-trioxo-hexahydro-pyrimidin-5-yl)-1,4-dimethyl-pyrrolidin (6e)

*N*-Cbz-Methylaminoacetaldehyd (25.9 mg, 125 µmol), *N,N*-Dimethylbarbitursäure (19.5 mg, 125 µmol) und Benzylprop-1-enylether (148 mg, 1.00 mmol) wurden mit 0.5 ml Toluol, 0.1 ml Trimethylorthoformiat und wenigen Kristallen Ethylendiammoniumdiacetat (EDDA) unter Argonatmosphäre in einem 10 ml-Druckkolben gemäß der AAV umgesetzt. Das Produkt wurde nach Lösen in Methanol und anschließendem Entfernen des Lösungsmittel als gelbliches Öl erhalten, welches später amorph auskristallisierte. Die per HPLC bestimmte Reinheit betrug >95 %.

^{**1**}**H-NMR** (300 MHz, DMSO): δ = 0.74, 0.95 (2d, *J*= 6.5 Hz, 3H, 1"-H), 2.52 - 2.68 (m, 2H, 4-H), 2.71, 2.75 (2s, 3H, NMe), 2.70 - 3.84 (3m, 5H, 2-H, 3-H, 5-H), 3.20, 3.40 (2s, 6H, MeNC(O)NMe).
**MS** (70eV): *m/z* = 253.2 (100) [M⁺], 97.1 (92) [M⁺ - C₆H₈N₂O₃], 82.1 (80) [M⁺ - C₆H₈N₂O₃ - Me].

**C**_{**12**}**H**_{**19**}**N**_{**3**}**O**_{**3**} **(253.1), HRMS: 253.1426.**

### 1-(1,3-Dimethyl-2,4,6-trioxo-hexahydro-pyrimidin-5-yl)-hexahydro-pyrrolizin (6f)

*N*-Cbz-Pyrrolidin-2-carbaldehyd (29.14 mg, 125 µmol), *N,N*-Dimethylbarbitursäure (19.5 mg, 125 µmol) und Benzylvinylether (134 mg, 1.00 mmol) wurden mit 0.5 ml Toluol, 0.1 ml Trimethylorthoformiat und wenigen Kristallen Ethylendiammoniumdiacetat (EDDA) unter Argonatmosphäre in einem 10 ml-Druckkolben gemäß der AAV umgesetzt. Das Produkt wurde nach Lösen in Methanol und anschließendem Entfernen des Lösungsmittel als gelbliches Öl erhalten, welches später amorph auskristallisierte.

^{**1**}**H-NMR** (500 MHz, DMSO-D₆): δ = 1.84 - 1.92, 1.98 - 2.08, 2.17 - 2.26, 2.44 - 2.54 (4m, 6H, 2-H, 6-H, 7-H), 3.04 - 3.11, 3.20 - 3.25, 3.37 - 3.58 (3m, 4H, 3-H, 5-H), 3.22 (s, 6H, NMe), 3.77 - 3.82 (m, 1H, 7a-H), 4.42 - 4.60 (m, 1H, 1-H).
^{**13**}**C-NMR** (50 MHz, CD₃OD): δ =24.88, 24.64, 30.19, 30.64, 30.97 (C-2, C-6, C-7), 27.74, 27.97, 28.10, 28.24 (NMe), 42.36, 42.07 (C-1), 52.94, 55.65, 56.35 (C-3, C-5), 68.63, 68.81, 70.45 (C-7a), 81.34, 84.94, 85.25 (C-5'), 154.7, 154.8 (C-2'), 165.2, 165.3 (C-4', C-6').
**MS** (70eV): *m/z* = 265.2 (10) [M⁺], 156.1 (45) [C₆H₈N₂O₃], 109.1 (73) [M⁺ - C₆H₈N₂O₃].

**C**_{**13**}**H**_{**19**}**N**_{**3**}**O**_{**3**} **(265.2).**

### 3-(1,3-Dimethyl-2,4,6-trioxo-hexahydro-pyrimidin-5-yl)-2,4-dimethyl-pyrrolidin (6g)

*N*-Cbz-2-Aminopropionaldehyd (25.9 mg, 125 µmol), *N,N*-Dimethylbarbitursäure (19.5 mg, 125 µmol) und Benzylprop-1-enylether (148 mg, 1.00 mmol) wurden mit 0.5 ml Toluol, 0.1 ml Trimethylorthoformiat und wenigen Kristallen Ethylendiammoniumdiacetat (EDDA) unter Argonatmosphäre in einem 10 ml-Druckkolben gemäß der AAV umgesetzt. Das Produkt wurde nach Lösen in Methanol und anschließendem Entfernen des Lösungsmittel als gelbliches Öl erhalten, welches später amorph auskristallisierte. Die per HPLC bestimmte Reinheit betrug 97 %.

^{**1**}**H-NMR** (500 MHz, DMSO-D₆): δ = 0.74, 0.85 (2d, *J*= 7 Hz, 3H, 1"'-H), 1.11, 1.19 (2d, *J*= 7 Hz, 3H, 1"-H), 2.48 - 2.56 (m, 1H, 4-H), 2.64 - 2.94 (3m, 2H, 5-H), 3.02, 3.08 (m, 1H, 2-H), 3.30-3.38 (m, 1H, 2-H), 3.90 - 3.98, 4.02 - 4.10 (2m, 1H, 3-H).
^{**13**}**C-NMR** (125 MHz, DMSO-D₆): δ = 14.33, 16.26, 16.68, 17.40 (1"-H, 1"'-H), 26.68, 26.94 (NMe), 33.72, 34.00, 44.43, 49.26 (C-3, C-4), 49.79, 50.40, (C-5), 56.10, 57.72 (C-2), 78.62, 81.78 (C-5'), 152.5, 152.8 (C-2'), 162.2, 162.7 (C-4', C-6').
**MS** (70eV): *m/z* = 253.2 (26) [M⁺], 238.2 (23) [M⁺ - Me], 97.1 (100) [M⁺ - C₆H₈N₂O₃], 82.1 (98) [M⁺ - C₆H₈N₂O₃ - Me].

**C**_{**12**}**H**_{**19**}**N**_{**3**}**O**_{**3**} **(253.1), HRMS: 253.1426.**

### 3-(1,3-Dimethyl-2,4,6-trioxo-hexahydro-pyrimidin-5-yl)-4-ethyl-2-methyl-pyrrolidin (6h)

*N*-Cbz-2-Aminopropionaldehyd (25.9 mg, 125 µmol), *N,N*-Dimethylbarbitursäure (19.5 mg, 125 µmol) und Benzylbut-1-enylether (162 mg, 1.00 mmol) wurden mit 0.5 ml Toluol, 0.1 ml Trimethylorthoformiat und wenigen Kristallen Ethylendiammoniumdiacetat (EDDA) unter Argonatmosphäre in einem 10 ml-Druckkolben gemäß der AAV umgesetzt. Das Produkt wurde nach Lösen in Methanol und anschließendem Entfernen des Lösungsmittel als gelbliches Öl erhalten, welches später amorph auskristallisierte. Die per HPLC bestimmte Reinheit betrug 97 %.

^{**13**}**C-NMR** (50 MHz, CD₃OD): δ = 12.62, 13.37 (C-2'''), 16.47, 17.90 (C-1"), 23.98, 27.28 (C-1'''), 227.99, 28.06, 28.24, 28.61 (NMe), 42.85, 43.28, 45.18, 48.86 (C-3, C-4), 60.35 (C-5), 58.55, 60.35 (C-2), 154.8, 154.9 (C-2'), 165.4, 165.6, 165.7, 165.8, 166.0 (C-4', C-6').
**MS** (70eV): *m/z* = 267.2 (8) [M⁺], 252.2 (6) [M⁺ - Me], 223.2 (1) [M⁺ - Me - Et], 111.1 (18) [M⁺ - C₆H₈N₂O₃].

**C**_{**13**}**H**_{**21**}**N**_{**3**}**O**_{**3**} **(267.2), HRMS: 267.1582.**

### 3-(1,3-Dimethyl-2,4,6-trioxo-hexahydro-pyrimidin-5-yl)-4-isopropyl-2-methyl-pyrrolidin (6i)

*N*-Cbz-2-Aminopropionaldehyd (25.9 mg, 125 µmol), *N,N*-Dimethylbarbitursäure (19.5 mg, 125 µmol) und Benzyl-3-methylbut-1-enylether (190 mg, 1.00 mmol) wurden mit 0.5 ml Toluol, 0.1 ml Trimethylorthoformiat und wenigen Kristallen Ethylendiammoniumdiacetat (EDDA) unter Argonatmosphäre in einem 10 ml-Druckkolben gemäß der AAV umgesetzt. Das Produkt wurde nach Lösen in Methanol und anschließendem Entfernen des Lösungsmittel als gelbliches Öl erhalten, welches später amorph auskristallisierte. Die per HPLC bestimmte Reinheit betrug 75 %.

^{**1**}**H-NMR** (500 MHz, DMSO-D₆): δ = 0.85 - 1.20 (mehrere d, 9H, *J* = 7 - 8 Hz, 1"-H, 2"'-H, 3"'-H), 1.30 - 1.56 (2m, 1H, 1"'-H), 1.85 - 2.15 (2m, 1H, 4-H), 3.00 - 3.80 (mehrere m, 10H, 2xNMe, 5-H, 2-H, 3-H).
^{**13**}**C-NMR** (125 MHz, DMSO-D₆): δ = 13.05, 13.46, 16.33, 18.13, 19.21, 20.22, 20.91, 21.49, 21.66, 22.06, 22.50, 22.73, 24.39, 26.77, 26.87, 26.99, 27.19, 27.23, 27.29, 27.45, 28.14, 29.34, 32.08 (-Me, -*i*Pr, NMe), 42.33, 47.71 (C-3, C-4), 48.04, 48.55 (C-5), 68.57, 60.12 (C-2), 83.46, 86.47 (C-5'), 152.5 (C-2'), 162.8, 162.9 (C-4', C-6').
**MS** (70eV): *m/z* = 281.2 (69) [M⁺], 266.2 (24) [M⁺ - Me], 239.1 (86) [M⁺ - *i*Pr + H].

**C**_{**14**}**H**_{**23**}**N**_{**3**}**O**_{**3**} **(281.2), HRMS: 281.1739.**

### 3-(1,3-Dimethyl-2,4,6-trioxo-hexahydro-pyrimidin-5-yl)-2-isopropyl-pyrrolidin (6k)

*N*-Cbz-2-Amino-3-methylbutyraldehyd (29.4 mg, 125 µmol), *N,N*-Dimethylbarbitursäure (19.5 mg, 125 µmol) und Benzylvinylether (134 mg, 1.00 mmol) wurden mit 0.5 ml Toluol, 0.1 ml Trimethylorthoformiat und wenigen Kristallen Ethylendiammoniumdiacetat (EDDA) unter Argonatmosphäre in einem 10 ml-Druckkolben gemäß der AAV umgesetzt. Das Produkt wurde nach Lösen in Methanol und anschließendem Entfernen des Lösungsmittel als gelbliches Öl erhalten, welches später amorph auskristallisierte. Die per HPLC bestimmte Reinheit betrug 95 %.

^{**1**}**H-NMR** (300 MHz, MeOH-D₄): δ = 0.82 - 1.10 (2d, *J*= 6 Hz, 6H, 2"-H, 3"-H), 1.80 - 2.30 (m, 3H, 4-H, 1"-H), 3.24 (s, 6H, NMe), 3.20 - 3.58 (2m, 3H, 5-H, 2-H), 3.75 - 3.82 (m, 1H, 3-H).
**MS** (70eV): *m/z* = 267.2 (1) [M⁺], 224.1 (100) [M⁺ - *i*Pr], 111.1 (6) [M⁺ - C₆H₈N₂O₃].

**C**_{**13**}**H**_{**21**}**N**_{**3**}**O**_{**3**} **(267.2).**

### 3-(1,3-Dimethyl-2,4,6-trioxo-hexahydro-pyrimidin-5-yl)-2-isopropyl-4-methyl-pyrrolidin (6I)

*N*-Cbz-2-Amino-3-methylbutyraldehyd (29.4 mg, 125 µmol), *N,N*-Dimethylbarbitursäure (19.5 mg, 125 µmol) und Benzylprop-l-enylether (148 mg, 1.00 mmol) wurden mit 0.5 ml Toluol, 0.1 ml Trimethylorthoformiat und wenigen Kristallen Ethylendiammoniumdiacetat (EDDA) unter Argonatmosphäre in einem 10 ml-Druckkolben gemäß der AAV umgesetzt. Das Produkt wurde nach Lösen in Methanol und anschließendem Entfernen des Lösungsmittel als gelbliches Öl erhalten, welches später amorph auskristallisierte. Die per HPLC bestimmte Reinheit betrug 78 %.

^{**13**}**C-NMR** (125 MHz, DMSO-D₆): δ = 12.90, 14.96, 16.18, 18.96, 19.39, 19.64, 20.28, 20.39, 26.83, 27.31, 27.64, 29.71, 31.14, 34.93, 35.19, 35.48, 39.11, 39.76, 40.66, 45.11 (-Me, -*i*Pr, NMe) 50.33, 50.83, 51.73 (C-5), 65.43, 68.39, 69.44 (C-2), 80.11, 80.51, 82.82 (C-5'), 152.8 (C-2'), 162.9, 163.4, 163.8 (C-4', C-6').
**MS** (70eV): *m/z* = 281.2 (10) [M⁺], 238.1 (100) [M⁺ - *i*Pr], 223.1 (1) [M⁺ - *i*Pr - Me].

**C**_{**14**}**H**_{**23**}**N**_{**3**}**O**_{**3**} **(281.2), HRMS: 281.1739.**

### 3-(1,3-Dimethyl-2,4,6-trioxo-hexahydro-pyrimidin-5-yl)-2,4-diisopropyl-pyrrolidin (6m)

*N*-Cbz-2-Amino-3-methylbutyraldehyd (29.4 mg, 125 µmol), *N,N-*Dimethylbarbitursäure (19.5 mg, 125 µmol) und Benzyl-3-methylbut-1-enylether (190 mg, 1.00 mmol) wurden mit 0.5 ml Toluol, 0.1 ml Trimethylorthoformiat und wenigen Kristallen Ethylendiammoniumdiacetat (EDDA) unter Argonatmosphäre in einem 10 ml-Druckkolben gemäß der AAV umgesetzt. Das Produkt wurde nach Lösen in Methanol und anschließendem Entfernen des Lösungsmittel als gelbliches Öl erhalten, welches später amorph auskristallisierte. Die per HPLC bestimmte Reinheit betrug 82 %.

^{**1**}**H-NMR** (500 MHz, DMSO-D₆): δ = 0.72 - 0.98 (mehrere d, *J*= 7 - 8 Hz, 12H, 2"-H, 3"-H, 2"'-H, 3"'-H), 1.30 - 1.40 (m, 1H, 1"'-H), 1.48 - 1.82 (2m, 2H, 4-H, 1"H), 3.00 - 3.12 (m, 8H, 2 x NMe, 5-H), 3.32 - 3.58 (2m, 1H, 2-H), 3.72 - 3.88 (2m, 1H, 3-H).
^{**13**}**C-NMR** (125 MHz, DMSO-D₆): δ = 19.27, 21.87, 20.06, 20.18, 20.25, 20.40, 20.90, 21.47, 21.71, 22.10, 22.59, 26.75, 27.12, 27.28, 27.32, 27.49, 27.55, 28.02, 29.40, 31.66, 32.14 (2 x -*i*Pr, NMe) 39.67, 39.93, 40.10, (C-3, C-4), 48.55, 48.95, 49.90 (C-3, C-4), 49.04, 49.96, 51.90 (C-5), 68.26, 70.01, 70.15 (C-2), 80.26, 84.10, 87.26 (C-5'), 152.4, 152.8 (C-2'), 162.4, 162.5, 163.4, 163.5, 163.7 (C-4', C-6').
**MS** (70eV): *m/z* = 309.2 (21) [M⁺], 266.2 (100) [M⁺ - *i*Pr], 110.1 (25) [M⁺ - C₆H₈N₂O₃ - *i*Pr].

**C**_{**16**}**H**_{**27**}**N**_{**3**}**O**_{**3**} **(309.2), HRMS: 309.2052.**

### 3-(1,3-Dimethyl-2,4,6-trioxo-hexahydro-pyrimidin-5-yl)-2-benzyl-pyrrolidin (6n)

N-Cbz-2-Amino-3-phenylpropionaldehyd (35.4 mg, 125 µmol), *N,N*-Dimethylbarbitursäure (19.5 mg, 125 µmol) und Benzylbut-1-enylether (162 mg, 1.00 mmol) wurden mit 0.5 ml Toluol, 0.1 ml Trimethylorthoformiat und wenigen Kristallen Ethylendiammoniumdiacetat (EDDA) unter Argonatmosphäre in einem 10 ml-Druckkolben gemäß der AAV umgesetzt. Das Produkt wurde nach Lösen in Methanol und anschließendem Entfernen des Lösungsmittel als gelbliches Öl erhalten, welches später amorph auskristallisierte. Die per HPLC bestimmte Reinheit betrug 97 %.

^{**1**}**H-NMR** (300 MHz, CD₃OD): δ = 2.05 - 2.45 (2m, 2H, 4-H), 2.90 - 2.95 (m, 2H, PhCH₂), 3.20 (s, 6H, 2NMe), 3.30 - 3.55 (m, 3H, 2-H, 5-H), 4.22 - 4.32 (m, 1H, 3-H), 7.15 - 7.30 (m, 5H, PhH).
^{**13**}**C-NMR** (75MHz, DMSO-D₆): δ = 26.17 (NMe), 27.21 (C-4), 39.87 (C-3), 35.28, 43.49 (C-1", C-5), 61.83 (C-2), 81.15 (C-5'), 125.5, 128.3, 128.7 (C-3", C-4", C-5", C-6") 131.0 (C-2"), 152.6 (C-2'), 162.0 (C-4', C-6').
**MS** (70eV): *m/z* = 315.2 (1) [M⁺], 224.1 (100) [M⁺ - Bn], 91.0 (10) [Bn].

**C**_{**15**}**H**_{**21**}**N**_{**3**}**O**_{**3**} **(315.2), HRMS: 315.1582.**

### 3-(1,3-Dimethyl-2,4,6-trioxo-hexahydro-pyrimidin-5-yl)-2-isobutyl-pyrrolidin (6o)

N-Cbz-2-Amino-4-methylpentanal (31.2 mg, 125 µmol), *N,N-*Dimethylbarbitursäure (19.5 mg, 125 µmol) und Benzylvinylether (134 mg, 1.00 mmol) wurden mit 0.5 ml Toluol, 0.1 ml Trimethylorthoformiat und wenigen Kristallen Ethylendiammoniumdiacetat (EDDA) unter Argonatmosphäre in einem 10 ml-Druckkolben gemäß der AAV umgesetzt. Das Produkt wurde nach Lösen in Methanol und anschließendem Entfernen des Lösungsmittel als gelbliches Öl erhalten, welches später amorph auskristallisierte. Die per HPLC bestimmte Reinheit betrug 99 %.

^{**1**}**H-NMR** (500 MHz, DMSO-D₆): δ = 0.88 (d, *J*= 8 Hz, 6H, 3"-H, 4"-H), 1.44 - 1.57, 1.60 - 1.70 (3H, 2"-H, 1"-H), 2.04 - 2.37 (2m, 2H, 4.H), 3.14 (s, 6H, NMe), 3.16 - 3.47 (2m, 3H, 2-H, 5-H), 3.98 - 4.03 (m, 1H, 3-H).
^{**13**}**C-NMR** (50 MHz, CD₃OD): δ = 22.38, 23.24, 26.80, 27.99, (C-2", C-3", C-4", NMe), 28.60 (C-4), 41.57 (C-3), 41.97, 45.59 (C-1", C-5), 60.72 (C-2), 86.10 (C-5'), 154.7 (C-2'), 165.1 (C-4', C-6').

**C**_{**14**}**H**_{**23**}**N**_{**3**}**O**_{**3**} **(281.2), HRMS: 281.1739.**

### 3-(1,3-Dimethyl-2,4,6-trioxo-hexahydro-pyrimidin-5-yl)-2-isobutyl-4-methyl-pyrrolidin (6p)

N-Cbz-2-Amino-4-methylpentanal (31.2 mg, 125 µmol), *N,N*-Dimethylbarbitursäure (19.5 mg, 125 µmol) und Benzylprop-1-enylether (148 mg, 1.00 mmol) wurden mit 0.5 ml Toluol, 0.1 ml Trimethylorthoformiat und wenigen Kristallen Ethylendiammoniumdiacetat (EDDA) unter Argonatmosphäre in einem 10 ml-Druckkolben gemäß der AAV umgesetzt. Das Produkt wurde nach Lösen in Methanol und anschließendem Entfernen des Lösungsmittel als gelbliches Öl erhalten, welches später amorph auskristallisierte. Die per HPLC bestimmte Reinheit betrug >95 %.

^{**1**}**H-NMR** (500 MHz, DMSO-D₆): δ = 0.75 - 0.88 (mehrere d, *J*= 7 Hz, 9H, 3"-H, 4"-H, 1"'-H), 1.35 - 1.65 (3m, 3H, 1"-H, 2"-H), 2.38 - 2.50 (m, 1H, 4-H), 3.00 - 3.40 (3m, 3H, 2-H, 5-H), 3.05, 3.08 (2s, 6H, NMe), 3.95-4.10 (2m, 1H, 3-H).
^{**13**}**C-NMR** (125 MHz, DMSO-D₆): δ = 13.79, 16.43 (C-1'''), 20.35, 21.81, 22.32, 22.38, 22.48, 22.90, 22.96, 24.76, 25.01, 25.04, 22.96, 22.99, 27.07, 27.14, 27.20, 27.26 (C-3", C-4", C-2", NMe), 33.94, 34.39, 42.68, 43.02 (C-3, C-4), 40.69, 41.49 (C-1"), 49.99, 51.09, 51.41 (C-5), 59.22, 60.01 (C-2), 79.09, 81.84 (C-5'), 152.9 (C-2'), 162.4 (C-4'. C-6').
**MS** (70eV): *m/z* = 295.2 (11) [M⁺], 238.1 (100) [M⁺ - *i*Bu], 139.1 (30) [M⁺ - C₆H₈N₂O₃].

**C**_{**15**}**H**_{**25**}**N**_{**3**}**O**_{**3**} **(295.2), HRMS: 295.1895.**

### 3-(1,3-Dimethyl-2,4,6-trioxo-hexahydro-pyrimidin-5-yl)-4-ethyl-2-isobutyl-pyrrolidin (6q)

N-Cbz-2-Amino-4-methylpentanal (31.2 mg, 125 µmol), *N,N*-Dimethylbarbitursäure (19.5 mg, 125 µmol) und Benzylbut-1-enylether (162 mg, 1.00 mmol) wurden mit 0.5 ml Toluol, 0.1 ml Trimethylorthoformiat und wenigen Kristallen Ethylendiammoniumdiacetat (EDDA) unter Argonatmosphäre in einem 10 ml-Druckkolben gemäß der AAV umgesetzt. Das Produkt wurde nach Lösen in Methanol und anschließendem Entfernen des Lösungsmittel als gelbliches Öl erhalten, welches später amorph auskristallisierte. Die per HPLC bestimmte Reinheit betrug >95 %.

^{**1**}**H-NMR** (500 MHz, DMSO-D₆): δ = 0.84-0.95 (mehrere d, *J*= 6.5 Hz, 9H, 2"'-H, 3"-H, 4"-H), 1.19-1.72 (5m, 5H, 1"-H, 2"-H, 1"'-H), 2.32 - 2.42 (m, 1H, 4-H), 3.22 - 3.26 (mehrere s, 6H, NMe), 3.30 - 3.43 (2m, 2H, 5-H), 3.48 - 3.60 (2m, 1H, 2-H), 4.05 - 4.17 (2m, 1H, 3-H).
^{**13**}**C-NMR** (50 MHz, CD₃OD): δ = 12.62, 13.43 (C-2"), 23.42, 26.93 (C-1'''), 22.76, 22.80, 22.68, 26.76, 27.94, 28.00, 28.45 (C-3", C-4", C-2", NMe), 42.17, 43.15 (C-1"), 42.80, 43.42, 43.76 (C-3, C-4), 50.41, 51.59 (C-5), 60.73, 62.59 (C-2), 86.54 (C-5'), 154.7 (C-2'), 154.4, 165.9 (C-4', C-6').
**MS** (70eV): *m/z* = 309.2 (16) [M⁺], 252.2 (100) [M⁺ - *i*Bu].

**C**_{**16**}**H**_{**27**}**N**_{**3**}**O**_{**3**} **(309.2), HRMS: 309.2052.**

### 3-(1,3-Dimethyl-2,4,6-trioxo-hexahydro-pyrimidin-5-yl)-2-isobutyl-4-isopropyl-pyrrolidin (6r)

N-Cbz-2-Amino-4-methylpentanal (31.2 mg, 125 µmol), *N,N*-Dimethylbarbitursäure (19.5 mg, 125 µmol) und Benzyl-3-methylbut-1-enylether (190 mg, 1.00 mmol) wurden mit 0.5 ml Toluol, 0.1 ml Trimethylorthoformiat und wenigen Kristallen Ethylendiammoniumdiacetat (EDDA) unter Argonatmosphäre in einem 10 ml-Druckkolben gemäß der AAV umgesetzt. Das Produkt wurde nach Lösen in Methanol und anschließendem Entfernen des Lösungsmittel als gelbliches Öl erhalten, welches später amorph auskristallisierte.
^{**13**}**C-NMR** (75 MHz, DMSO-D₆): δ = 21.67, 22.11, 22.44, 22.52, 24.70, 24.88, 26.77, 27.04, 27.25, 27.75 (2 x -*i*Pr, NMe), 42.20 (C-1"), 40.78, 48.33 (C-3, C-4), 49.33 (C-5), 59.69, 62.20 (C-2), 83.54 (C-5'), 152.3, 152.6, 152.8 (C-2'), 161.8, 162.5, 162.6, 162.6 (C-4', C-6').
**MS** (70eV): *m/z* = 323.2 (22) [M⁺], 266.2 (100) [M⁺ - *i*Bu].

**C**_{**17**}**H**_{**29**}**N**_{**3**}**O**_{**3**} **(323.2), HRMS: 323.2208.**

### 4-(1,3-Dimethyl-2,4,6-trioxo-hexahydro-pyrimidin-5-yl)-azepan (8a)

N-Cbz-Pyrrolidin-2-ol (27.6 mg, 125 µmol), N,N-Dimethylbarbitursäure (19.5 mg, 125 µmol) und Benzylvinylether (134 mg, 1.00 mmol) wurden mit 0.5 ml Toluol, 0.1 ml Trimethylorthoformiat und wenigen Kristallen Ethylendiammoniumdiacetat (EDDA) unter Argonatmosphäre in einem 10 ml-Druckkolben gemäß der AAV umgesetzt. Das Produkt wurde nach Lösen in Methanol und anschließendem Entfernen des Lösungsmittel als gelbliches Öl erhalten, welches später amorph auskristallisierte. Die per HPLC bestimmte Reinheit betrug 98 %.

^{**13**}**C-NMR** (125 MHz, DMSO): δ = 22.80, 23.54, 29.89, 29.93 (C-3, C-4, C-6), 26.82, 26.88, 26.90, 27.04, 32.00 (NMe, C-5), 46.54, 46.60, (C-2, C-7), 89.50, (C-5'), 152.5 (C-2'), 162.1 (C-4'), 162.1 (C-4', C-6').

**C**_{**12**}**H**_{**19**}**N**_{**3**}**O**_{**3**} **(253.2).**

### 4-(1,3-Dimethyl-2,4,6-trioxo-hexahydro-pyrimidin-5-yl)-3-methyl-azepan (8b)

N-Cbz-Pyrrolidin-2-ol (27.6 mg, 125 µmol), N,N-Dimethylbarbitursäure (19.5 mg, 125 µmol) und Benzylprop-1-enylether (148 mg, 1.00 mmol) wurden mit 0.5 ml Toluol, 0.1 ml Trimethylorthoformiat und wenigen Kristallen Ethylendiammoniumdiacetat (EDDA) unter Argonatmosphäre in einem 10 ml-Druckkolben gemäß der AAV umgesetzt. Das Produkt wurde nach Lösen in Methanol und anschließendem Entfernen des Lösungsmittel als gelbliches Öl erhalten, welches später amorph auskristallisierte. Die per HPLC bestimmte Reinheit betrug >95 %.
^{**13**}**C-NMR** (125 MHz, DMSO): δ = 21.15, 23.16, 26.96, 27.23, 27.44, 32.89 (C-3, C-4, C-5, C-6, C-1", NMe), 48.64, 53.30 (C-2, C-7), 88.14 (C-5'), 152.4 (C-2'), 162.5 (C-4', C-6').

**C**_{**13**}**H**_{**21**}**N**_{**3**}**O**_{**3**} **(267.2).**

### 3-(2,4-Dioxo-chroman-3-yl)-2-methyl-pyrrolidin (6s)

N-Cbz-2-Aminopropionaldehyd (25.9 mg, 125 µmol), 4-Hydroxycumarin (20.25 mg, 125 µmol) und Benzylvinylether (134 mg, 1.00 mmol) wurden mit 0.5 ml Toluol, 0.1 ml Trimethylorthoformiat und wenigen Kristallen Ethylendiammoniumdiacetat (EDDA) unter Argonatmosphäre in einem 10 ml-Druckkolben gemäß der AAV umgesetzt. Das Produkt wurde nach Lösen in Methanol und anschließendem Entfernen des Lösungsmittel als gelbliches Öl erhalten, welches später amorph auskristallisierte. Die per HPLC bestimmte Reinheit betrug 95.0 %.

^{**1**}**H-NMR** (500 MHz, DMSO-D₆): δ = 1.10, 1.14 (2d, *J*= 6 Hz, 3H, 1"-H), 2.00 - 2.20 (2m, 2H, 4-H), 3.15-3.24, 3.30 - 3.45 (2m, 3H, 2-H, 5-H), 3.72 - 3.80, 3.88 - 3.95 (2m, 1H, 3-H), 7.02 - 7.14 (m, 2H, 6'-H, 7'-H), 7.30 - 7.41, 7.74 - 7.82 (2m, 2H, 5'-H, 8'-H).

**C**_{**14**}**H**_{**15**}**NO**_{**3**} **(245.1), HRMS: 245.1051.**

### 4-(2,4-Dioxo-chroman-3-yl)-piperidin (7h)

*N*-Cbz-3-Aminopropionaldehyd (25.9 mg, 125 µmol), 4-Hydroxycumarin (20.25 mg, 125 µmol) und Benzylvinylether (134 mg, 1.00 mmol) wurden mit 0.5 ml Toluol, 0.1 ml Trimethylorthoformiat und wenigen Kristallen Ethylendiammoniumdiacetat (EDDA) unter Argonatmosphäre in einem 10 ml-Druckkolben gemäß der AAV umgesetzt. Das Produkt wurde nach Lösen in Methanol und anschließendem Entfemen des Lösungsmittel als gelbliches Öl erhalten, welches später amorph auskristallisierte. Die per HPLC bestimmte Reinheit betrug >95 %.

^{**13**}**C-NMR** (125 MHz, DMSO-D₆): δ = 25.65 (C-3, C-5), 30.80 (C-4), 44.25 (C-2, C-6), 102.9 (C-3'), 115.7, 122.7, 124.4, 130.7 (C-5', C-6', C-7', C-8'), 120.0 (C-4a), 152.9 (C-8a), 162.9, 166.7 (C-2', C-4').

**C**_{**14**}**H**_{**15**}**NO**_{**3**} **(245.1), HRMS: 245.1051.**

### 3-(3,3-Dimethyl-2,6-dioxo-cyclohexyl)-2-methyl-pyrrolidin (6t)

*N*-Cbz-2-Aminopropionaldehyd (25.9 mg, 125 µmol), 4,4-Dimethylcyclohexan-1,3-dion (17.5 mg, 125 µmol) und Benzylvinylether (134 mg, 1.00 mmol) wurden mit 0.5 ml Toluol, 0.1 ml Trimethylorthoformiat und wenigen Kristallen Ethylendiammoniumdiacetat (EDDA) unter Argonatmosphäre in einem 10 ml-Druckkolben gemäß der AAV umgesetzt. Das Produkt wurde nach Lösen in Methanol und anschließendem Entfernen des Lösungsmittel als gelbliches Öl erhalten, welches später amorph auskristallisierte. Die per HPLC bestimmte Reinheit betrug >95 %.

^{**1**}**H-NMR** (300 MHz, DMSO-D₆): δ = 0.92 (s, 6H, C(CH₃)₂), 1.00 - 1.05 (m, 3H, 1"-H), 1.57 (t, *J*= 5.5 Hz, 2H, 5'-H), 1.62 - 2.05 (m, 2H, 4-H), 2.15 (t, *J*= 5.5 Hz, 2H, 6'-H), 3.00 - 3.42 (3m, 3H, 2-H, 5-H), 3.60 (m, 1H, 3-H).

**C**_{**13**}**H**_{**21**}**NO**_{**2**} **(223.2).**

### 3-(4,4-Dimethyl-2,6-dioxo-cyclohexyl)-2-methyl-pyrrolidin (6u)

*N*-Cbz-2-Aminopropionaldehyd (25.9 mg, 125 µmol), 5,5-Dimethylcyclohexan-1,3-dion (17.5 mg, 125 µmol) und Benzylvinylether (134 mg, 1.00 mmol) wurden mit 0.5ml Toluol, 0.1 ml Trimethylorthoformiat und wenigen Kristallen Ethylendiammoniumdiacetat (EDDA) unter Argonatmosphäre in einem 10 ml-Druckkolben gemäß der AAV umgesetzt. Das Produkt wurde nach Lösen in Methanol und anschließendem Entfernen des Lösungsmittel als gelbliches Öl erhalten, welches später amorph auskristallisierte. Die per HPLC bestimmte Reinheit betrug >95 %.

^{**1**}**H-NMR** (300 MHz, DMSO-D₆): δ = 0.86, 0.87 (2s, 6H, C(CH₃)₂), 0.95, 1.05 (2d, *J* = 6 Hz, 3H, 1"-H), 1.78 - 2.22 (m, 2H, 4-H), 1.96, 1.97 (2s, 4H, 3'-H, 5'-H), 2.78 - 3.30 (m, 3H, 2-H, 5-H), 3.66 - 3.80 (m, 1H, 3-H).

**C**_{**13**}**H**_{**21**}**NO**_{**2**} **(223.2).**

### 4-(1,3-Dimethyl-2,4,6-trioxo-hexahydro-pyrimidin-5-yl)-piperidin (7a)

*N*-Cbz-3-Aminopropionaldehyd (25.9 mg, 125µmol), *N,N*-Dimethylbarbitursäure (19.5 mg, 125 µmol) und Benzylvinylether (134 mg, 1.00 mmol) wurden mit 0.5 ml Toluol, 0.1 ml Trimethylorthoformiat und wenigen Kristallen Ethylendiammoniumdiacetat (EDDA) unter Argonatmosphäre in einem 10 ml-Druckkolben gemäß der AAV umgesetzt. Das Produkt wurde nach Lösen in Methanol und anschließendem Entfernen des Lösungsmittel als gelbliches Öl erhalten, welches später amorph auskristallisierte. Die per HPLC bestimmte Reinheit betrug >95 %.

^{**13**}**C-NMR** (50 MHz, CD₃OD): δ = 22.55 (C-3, C-5), 28.02 (NMe), 32.74 (C-4), 46.53 (C-2, C-6), 91.72 (C-5'), 154.7 (C-2'), 165.1 (C-4', C-6').
**MS** (70eV): *m/z* = 239.2 (37) [M⁺], 156.1 (48) [C₆H₈N₂O₃], 83.0 (100) [M⁺ - C₆H₈N₂O₃].

**C**_{**11**}**H**_{**17**}**N**_{**3**}**O**_{**3**} **(239.2).**

### 4-(1,3-Dimethyl-2,4,6-trioxo-hexahydro-pyrimidin-5-yl)-5-methyl-piperidin (7b)

*N*-Cbz-3-Aminopropionaldehyd (25.9 mg, 125µmol), *N,N*-Dimethylbarbitursäure (19.5 mg, 125 µmol) und Benzylprop-1-enylether (148 mg, 1.00 mmol) wurden mit 0.5 ml Toluol, 0.1 ml Trimethylorthoformiat und wenigen Kristallen Ethylendiammoniumdiacetat (EDDA) unter Argonatmosphäre in einem 10 ml-Druckkolben gemäß der AAV umgesetzt. Das Produkt wurde nach Lösen in Methanol und anschließendem Entfernen des Lösungsmittel als gelbliches Öl erhalten, welches später amorph auskristallisierte.

**MS** (70eV): *m/z =* 253.2 (18) [M⁺], 238.1 (5) [M⁺ - Me], 98.1 (100) [M⁺ - C₆H₈N₂O₃].

**C**_{**12**}**H**_{**19**}**N**_{**3**}**O**_{**3**} **(253.1), HRMS: 253.1426.**

### 4-(1,3-Dimethyl-2,4,6-trioxo-hexahydro-pyrimidin-5-yl)-5-ethyl-piperidin (7c)

*N*-Cbz-3-Amino-propionaldehyd (25.9 mg, 125µmol), *N,N*-Dimethylbarbitursäure (19.5 mg, 125 µmol) und Benzylbut-1-enylether (162 mg, 1.00 mmol) wurden mit 0.5 ml Toluol, 0.1 ml Trimethylorthoformiat und wenigen Kristallen Ethylendiammoniumdiacetat (EDDA) unter Argonatmosphäre in einem 10 ml-Druckkolben gemäß der AAV umgesetzt. Das Produkt wurde nach Lösen in Methanol und anschließendem Entfernen des Lösungsmittel als gelbliches Öl erhalten, welches später amorph auskristallisierte.

**MS** (70eV): *m/z* = 267.1 (30) [M⁺], 238.1 (18) [M⁺ - Et], 156.1 (38) [C₆H₈N₂O₃], 112.1 (89) [M⁺ - C₆H₈N₂O₃].

**C**_{**13**}**H**_{**21**}**N**_{**3**}**O**_{**3**} **(267.2), HRMS: 267.1582.**

### 4-(1,3-Dimethyl-2,4,6-trioxo-hexahydro-pyrimidin-5-yl)-5-isopropyl-piperidin (7d)

*N*-Cbz-3-Aminopropionaldehyd (25.9 mg, 125µmol), *N,N*-Dimethylbarbitursäure (19.5 mg, 125 µmol) und Benzyl-3-methylbut-1-enylether (190 mg, 1.00 mmol) wurden mit 0.5 ml Toluol, 0.1 ml Trimethylorthoformiat und wenigen Kristallen Ethylendiammoniumdiacetat (EDDA) unter Argonatmosphäre in einem 10 ml-Druckkolben gemäß der AAV umgesetzt. Das Produkt wurde nach Lösen in Methanol und anschließendem Entfernen des Lösungsmittel als gelbliches Öl erhalten, welches später amorph auskristallisierte.

**MS** (70eV): *m/z* = 281.1(33) [M⁺], 238.1 (20) [M⁺ - *i*Pr], 156.0 (100) [C₆H₈N₂O₃], 126.1 (55) [M⁺ - C₆H₈N₂O₃].

**C**_{**14**}**H**_{**23**}**N**_{**3**}**O**_{**3**} **(281.2), HRMS: 281.1817.**

### 4-(1,3-Dimethyl-2,4,6-trioxo-hexahydro-pyrimidin-5-yl)-2,5-dimethyl-piperidin (7e)

*N*-Cbz-3-Benzylaminobutyraldehyd (38.9 mg, 125µmol), *N,N*-Dimethylbarbitursäure (19.5 mg, 125 µmol) und Benzylprop-1-enylether (148 mg, 1.00 mmol) wurden mit 0.5 ml Toluol, 0.1 ml Trimethylorthoformiat und wenigen Kristallen Ethylendiammoniumdiacetat (EDDA) unter Argonatmosphäre in einem 10 ml-Druckkolben gemäß der AAV umgesetzt. Das Produkt wurde nach Lösen in Methanol und anschließendem Entfernen des Lösungsmittel als gelbliches Öl erhalten, welches später amorph auskristallisierte. Die per HPLC bestimmte Reinheit betrug >95 %.
**MS** (70eV): *m/z* = 267.1 (30) [M⁺], 252.2 (33) [M⁺ - Me], 156.1 (7) [C₆H₈N₂O₃], 112.1 (100) [M⁺ - C₆H₈N₂O₃]. 96.1 (65) [M⁺ - C₆H₈N₂O₃ - Me].

**C**_{**13**}**H**_{**21**}**N**_{**3**}**O**_{**3**} **(267.2).**

### 1-Butyl-4-(1,3-Dimethyl-2,4,6-trioxo-hexahydro-pyrimidin-5-yl)-2-methyl-piperidin (7f)

*N*-Cbz-3-Butylaminobutyraldehyd (34.7 mg, 125µmol), *N,N*-Dimethylbarbitursäure (19.5 mg, 125 µmol) und Benzylvinylether (134 mg, 1.00 mmol) wurden mit 0.5 ml Toluol, 0.1 ml Trimethylorthoformiat und wenigen Kristallen Ethylendiammoniumdiacetat (EDDA) unter Argonatmosphäre in einem 10 ml-Druckkolben gemäß der AAV umgesetzt. Das Produkt wurde nach Lösen in Methanol und anschließendem Entfernen des Lösungsmittel als gelbliches Öl erhalten, welches später amorph auskristallisierte. Die per HPLC bestimmte Reinheit betrug >95 %.

**MS** (70eV): *m/z* = 309.3 (7) [M⁺], 294.3 (43) [M⁺ - Me], 266.7 (100) [M⁺ - C₃H₇], 138.2 [M⁺ - C₆H₈N₂O₃ - Me].

**C**_{**16**}**H**_{**27**}**N**_{**3**}**O**_{**3**} **(309.2), HRMS: 309.2052.**

### 1-Butyl-4-(1,3-Dimethyl-2,4,6-trioxo-hexahydro-pyrimidin-5-yl)-2,5-dimethyl-piperidin (7g)

*N*-Cbz-3-Butylaminobutyraldehyd (34.7 mg, 125µmol), *N,N-*Dimethylbarbitursäure (19.5 mg, 125 µmol) und Benzylprop-1-enylether (148 mg, 1.00 mmol) wurden mit 0.5 ml Toluol, 0.1 ml Trimethylorthoformiat und wenigen Kristallen Ethylendiammoniumdiacetat (EDDA) unter Argonatmosphäre in einem 10 ml-Druckkolben gemäß der AAV umgesetzt. Das Produkt wurde nach Lösen in Methanol und anschließendem Entfernen des Lösungsmittel als gelbliches Öl erhalten, welches später amorph auskristallisierte.

**MS** (70eV): *m/z* = 323.2 (8) [M⁺], 280.2 (100) [M⁺ - C₃H₇].

**C**_{**16**}**H**_{**27**}**N**_{**3**}**O**_{**3**} **(323.2), HRMS: 323.2208.**

## Patentansprüche

1. Verfahren zur kombinatorischen Herstellung einer Substanzbibliothek aus n verschiedenen Verbindungen in einem Array aus n verschiedenen Reaktionsansätzen an n räumlich getrennten Reaktionsorten, bei dem an jedem der n Reaktionsorte
a) aus jeweils m Reaktanten in einer Reaktion oder Reaktionssequenz eine zwitterionische Verbindung in einem polaren Lösungsmittel gelöst erhalten wird,
b) die zwitterionische Verbindung durch Zugabe eine unpolaren Lösungsmittels als Feststoff gefällt, isoliert und damit in reiner Form erhalten wird,
c) gegebenenfalls die zwitterionische Verbindung weiter umgesetzt wird, wobei sich jeder der n Reaktionsansätze von jedem der n - 1 übrigen Reaktionsansätzen in mindestens einem der m Reaktanten unterscheidet.

2. Verfahren nach Anspruch 1, **dadurch gekennzeichnet, daß** als m Reaktanten ein geschützter Aminoaldehyd der allgemeinen Formel 1, 2 oder 3 mit einem Enolether der allgemeinen Formel 5 und einer 1,3-Dicarbonylverbindung 4 in untenstehender Reaktionssequenz A zu einem Betain der allgemeinen Formel 6, 7 bzw. 8 als zwitterionischer Verbindung umgesetzt werden. 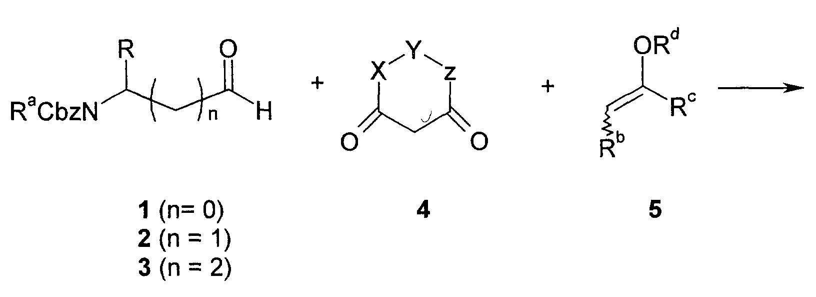 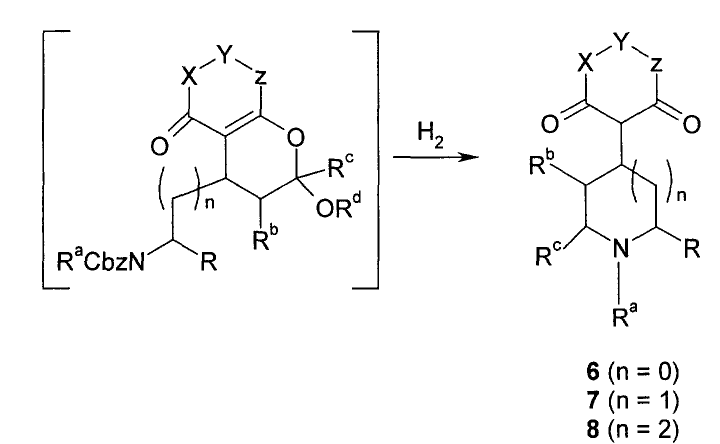 mit R^{d} = Benzyl oder Si(CH₃)₃.

3. Verfahren nach Anspruch 2, **dadurch gekennzeichnet, daß** der geschützte Aminoaldehyd ausgwählt ist aus den alpha-, beta- und gamma-Aminoaldehyden der allgemeinen Formeln 1a bis 1e: 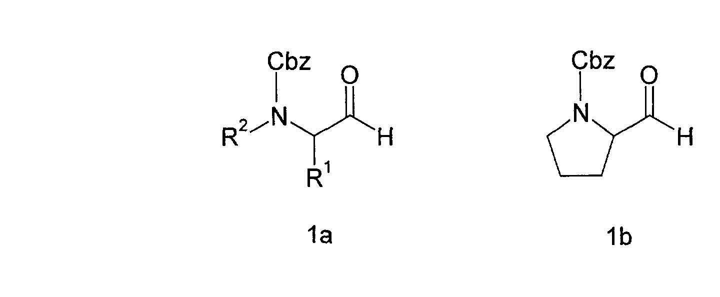 mit
R¹ = H, Alkyl, CH₂OH, CH₂SH,
R² = H, Alkyl,
und
Alkyl = Me, Et, n-Pr, i-Pr, n-Bu, sek-Bu, i-Bu oder Pentyl 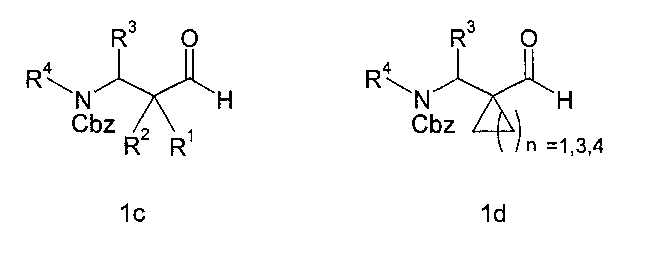 mit
R¹, R², gleich oder verschieden, H, Alkyl
R³, R⁴, gleich oder verschieden, H, Alkyl,
und Alkyl = Me, Et, n-Pr, i-Pr, n-Bu, sek-Bu, i-Bu oder Pentyl 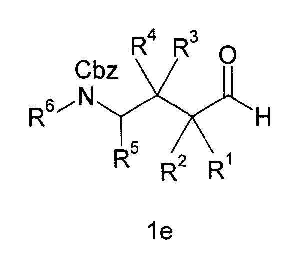 mit
R¹, R², R³, R⁴, gleich oder verschieden, H, Alkyl
R⁵, R⁶, gleich oder verschieden, H, Alkyl,
und Alkyl = Me, Et, n-Pr, n-Bu, sek-Bu, i-Bu oder Pentyl

4. Verfahren nach Anspruch 2 oder 3, **dadurch gekennzeichnet, daß** der Enolether ausgewählt ist aus den Enolethern der allgemeinen Formeln 2a bis 2d: 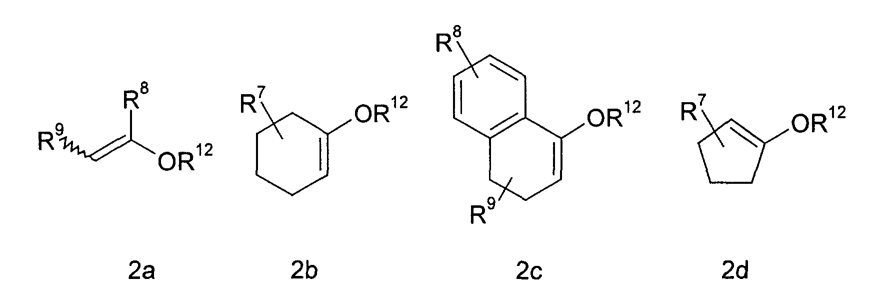 mit
R⁷, R⁸, R⁹, gleich oder verschieden, H, Alkyl, Bn, Ph-C₂H₄,CH₂OR, CH₂SR, CH₂NHAc, CH₂CH₂OR, CH₂CH₂SR, CH₂CH₂NHAc, und
R⁹ zusätzlich Ph,
R¹² Benzyl oder Si(CH₃)₃,
und Alkyl = Me, Et, n-Pr, i-Pr, n-Bu, sek-Bu, i-Bu oder Pentyl

5. Verfahren nach einem der Ansprüche 2 bis 4, **dadurch gekennzeichnet, daß** die 1,3-Dicarbonylverbindung ausgewählt ist aus den 1,3-Dicarbonylverbindungen der allgemeinen Formeln 3a und 3b: 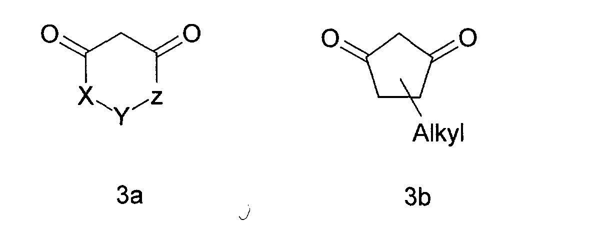 mit
X, Z, gleich oder verschieden, NH, NAlkyl, CH₂, CMe₂, O, S, und
Y CH₂, CMe₂, C=O, C=S,
oder
X-Y-Z NH-CR¹⁰=CR¹¹, NAlkyl-CR¹⁰=CR¹¹,
O-CR¹⁰=CR¹¹, NAlkyl-CHR¹-CHR²,
mit R¹⁰ und R¹¹, gleich oder verschieden, Alkyl, unsubstituiertes oder mit OH, OAlkyl, NH₂, NHAlkyl oder N(Alkyl)₂ substituiertes Phenyl, unsubstituiertes oder mit OH, Oalkyl, NH₂, NHAlkyl oder N(Alkyl)₂ substituiertes 1,3-Butadien-1,3-dienyl, wobei im zuletzt genannten Fall R¹⁰ und R¹¹ Teile des gleichen Ringsystems sind,
und Alkyl = Me, Et, n-Pr, i-Pr, n-Bu, sek-Bu, i-Bu oder Pentyl.

6. Verfahren nach einem der Ansprüche 1 bis 5, **dadurch gekennzeichnet, daß** die zwitterionische Verbindung in Wasser, Methanol oder deren Gemischen als polarem Lösungsmittel erhalten werden.

7. Verfahren nach einem der Ansprüche 1 bis 6, **dadurch gekennzeichnet, daß** die zwitterionische Verbindung durch Zugabe von Diethylether als unpolarem Lösungsmittel gefällt wird.
